# EUROPEAN PATENT APPLICATION

(11) **EP 2 522 736 A1**
(43) Date of publication of application: **14.11.2012**
(21) Application number: 11731824.6
(22) Date of filing: 06.01.2011
(51) Int. Cl.: C12P 13/02

(54) **PROCESS FOR PRODUCTION OF CARBAMATE COMPOUND**

(30) Priority: 18.05.2010 JP 2010113875; 08.01.2010 JP 2010002648
(71) Applicant: Ube Industries, Ltd., Ube-shi Yamaguchi 755-8633 (JP)
(72) Inventor: YAMAMOTO, Yasuhito, Ube-shi,Yamaguchi 755-8633 (JP); YOSHIDA, Yasutaka, Ube-shi,Yamaguchi 755-8633 (JP); ARAKI, Mayumi, Ube-shi,Yamaguchi 755-8633 (JP)
(74) Representative: HOFFMANN EITLE
(86) International application number: PCT/JP2011/050128
(87) International publication number: WO 2011/083826

(57) **Abstract**

There is provided a process for preparing a carbamate compound, which is easy and commercially advantageous in that a carbamate compound can be produced with high yield from an amine compound and a carbonate compound. A process for preparing a carbamate compound which comprises the step of reacting an amine compound which has at least one amino group per molecule wherein the amine compound is selected from the group consisting of an aliphatic amine which may be substituted by an alicyclic group or an aromatic group or which may be interrupted by an alicyclic group or an aromatic group, and an alicyclic amine which may be substituted by an aliphatic group, with a carbonate compound in the presence of at least one organic solvent selected from the group consisting of a saturated cyclic hydrocarbon, an unsaturated cyclic hydrocarbon, and a non-cyclic ether by using a hydrolase.

## Description

### TECHNICAL FIELD

The present invention relates to a process for preparing a carbamate compound by reacting a carbonate compound with an amine compound.

### BACKGROUND ART

Generally, a carbamate compound is a compound useful in various fields as a medical or agricultural chemical, an organic material, or a raw material and intermediate therefor. Particularly, the carbamate compound is useful, for example, as a raw material in the preparation of an isocyanate compound. Conventionally, as a process for preparing a carbamate compound without using toxic phosgene, it has been known a process using a solid catalyst, such as a metal salt (for example, see Patent Literatures 1 and 2).

It has been known a method in which a haloformate compound instead of phosgene is reacted with an amine compound to produce a carbamate compound (for example, non-Patent Literature 1).
It has been known a method in which an amine compound is reacted with a dialkyl carbonate in the presence of an alkali metal compound to produce a carbamate compound (for example, non-Patent Literatures 2 and 3).
Further, it has been known a method in which a dialkyl carbonate is reacted in the presence of an organic amine compound to produce a carbamate compound (for example, non-Patent Literature 4).
In the above method of non-Patent Literature 1, the formed halogen compound residue is mixed into the produced carbamate compound after the reaction, and when such a carbamate compound is used in an electric or electronic material, a serious problem in that the residue adversely affects the properties of the resultant material is likely to occur.
In the above method of non-Patent Literatures 2 and 3, the alkali metal compound residue is mixed into the produced carbamate compound, and a similar problem occurs when such a carbamate compound is used in an electric or electronic material.
Further, the above method of non-Patent Literature 4 has a problem in that an amine-like odor is mixed into the produced carbamate compound, causing the products to have an unpleasant odor.
Therefore, with respect to the removal of the above residues and the prevention of mixing of the residues into the produced carbamate compound, various studies, such as studies of the method for reaction, and modification of the process for production, are being currently conducted.

On the other hand, recently, a synthesis reaction using an enzyme has an advantage in that a stereoselective reaction can be made under mild conditions, and hence various examples of the syntheses using an enzyme have been reported. As a method for obtaining a carbamate compound using a hydrolase, there has been known a method for synthesizing a carbamate by a reaction of 3',5'-diaminonucleoside (amine compound) and diethyl carbonate in a mixed solvent of pyridine and tetrahydrofuran in the presence of a hydrolase (for example, non-Patent Literature 5). However, in this method, the enzyme is used in a large amount based on the substrate, and the reaction requires a prolonged period of time and the yield of the product is low, and thus this method is not a satisfactory production method from a commercial point of view.

There have been reported examples in which optical resolution is performed utilizing a carbamate formation reaction by a reaction of a racemic amine and a carbonate by using a hydrolase. However, in these examples, the enzyme is used in a large amount based on the substrate and the reaction requires a long period of time (for example, non-Patent Literatures 6 and 7).

There is an example in which an optically active amine is synthesized by enzymatic desymmetrization by reacting a diamine with allyl carbonate in 1,4-dioxane solvent in the presence of a hydrolase. However, also in this case, the catalyst is used in a large amount and the reaction requires a long period of time. Further, this example uses toxic 1,4-dioxane as a solvent and hence is not a commercially advantageous method (for example, non-Patent Literature 8). Furthermore, the method of non-Patent Literature 8 has a problem in that even when a diamine compound having two amino groups in the equivalent environment is used, only one of the amino groups in the diamine compound is converted into a carbamate group due to the stereoselectivity of the enzyme, so that only a monocarbamate compound can be obtained.

As mentioned above, examples of the syntheses of a carbamate by a reaction of an amine compound and a carbonate compound in the presence of a hydrolase catalyst have been known, but, in all the examples, the catalyst is used in a large amount based on the amine compound and carbonate compound as raw materials, and the reaction requires a long period of time and the yield of the product is low, and therefore an example of an efficient synthesis of carbamate has not been reported.

Patent Literature 1: JP S54-88201A
Patent Literature 2: JP 2004-262892A

Non-Patent Literature 1: Synthetic Communications, 2006, Vol. 36, p. 3537-3548
Non-Patent Literature 2: Synthetic Communications, 2005, Vol. 35, p. 2099-2105
Non-Patent Literature 3: Synthetic Communications, 1994, Vol. 24, p. 2441-2448
Non-Patent Literature 4: Can. J. Chem., 1991, Vol. 69, p. 2059-2063
Non-Patent Literature 5: J. Org. Chem., 2004, Vol. 69, 1748-1751
Non-Patent Literature 6: Tetrahedron, 1993, Vol. 49, 4321-4326
Non-Patent Literature 7: Tetrahedron, 2000, Vol. 56, 1387-1391
Non-Patent Literature 8: J. Org. Chem., 2009, Vol. 74, 2571-2574

### DISCLOSURE OF THE INVENTION

### PROBLEMS TO BE SOLVED BY THE INVENTION

An object of the present invention is to provide a process for preparing a carbamate compound, which is easy and commercially advantageous in that a carbamate compound can be produced with high yield from an amine compound and a carbonate compound.

### MEANS TO SOLVE THE PROBLEMS

The present inventors have found that a desired carbamate compound can be produced with high yield and that this method is commercially advantageous by reacting an amine compound with a carbonate compound in at least one organic solvent selected from a cyclic saturated hydrocarbon, a cyclic unsaturated hydrocarbon, and a non-cyclic ether in the presence of a hydrolase catalyst, whereby the present invention has been accomplished.

Specifically, the present invention relates to a process for preparing a carbamate compound which comprises the step of reacting an amine compound which has at least one amino group per molecule wherein the amine compound is selected from the group consisting of an aliphatic amine which may be substituted by an alicyclic group or an aromatic group or which may be interrupted by an alicyclic group or an aromatic group, and an alicyclic amine which may be substituted by an aliphatic group, with a carbonate compound in the presence of at least one organic solvent selected from the group consisting of a saturated cyclic hydrocarbon, an unsaturated cyclic hydrocarbon, and a non-cyclic ether by using a hydrolase
The present invention relates to the above-mentioned process for preparing a carbamate compound, wherein the organic solvent is at least one organic solvent selected from the group consisting of an unsubstituted cycloalkyl, an aromatic hydrocarbon, and a dialkyl ether.
The present invention relates to the above-mentioned process for preparing a carbamate compound, wherein the hydrolase is immobilized on a support.
The present invention relates to the above-mentioned method for producing a carbamate, wherein the hydrolase is a hydrolase immobilized on a support which is incorporated as a fixed bed to the inner side of a reaction vessel, and the reaction is a reaction comprising the step of allowing the amine compound and the carbonate compound to pass through the reaction vessel.
The present invention relates to the above-mentioned process for preparing a carbamate compound, wherein the hydrolase is a lipase.
The present invention relates to the above-mentioned process for preparing a carbamate compound, wherein the amine compound is a monoamine compound represented by the following formula (1):

R¹—(CH₂)ₙ—NH₂ (1)

wherein R¹ is a linear or branched alkyl group having 1 to 20 carbon atoms, a linear or branched alkenyl group having 2 to 20 carbon atoms, a linear or branched alkynyl group having 2 to 20 carbon atoms, a cycloalkylalkyl group having 4 to 24 carbon atoms, an aralkyl group having 7 to 21 carbon atoms, or a cycloalkyl group having 3 to 20 carbon atoms, each of which may have a substituent, and
n is 0 or 1.
The present invention relates to the above-mentioned process for preparing a carbamate compound, wherein the amine compound is a diamine compound represented by the following formula (4):

H₂N—(CH₂)ₘ—R³-(CH₂)_{P} —NH₂ (4)

wherein R³ is a linear or branched alkylene group having 1 to 30 carbon atoms, a (linear alkylene having 1 to 4 carbon atoms)-(cycloalkylene having 3 to 20 carbon atoms)-(linear alkylene having 1 to 4 carbon atoms) group, a (linear alkylene having 1 to 4 carbon atoms)-(arylene having 6 to 20 carbon atoms)-(linear alkylene having 1 to 4 carbon atoms) group, a cycloalkylene group having 3 to 20 carbon atoms, or a (linear alkylene having 1 to 4 carbon atoms)-(cycloalkylene having 3 to 20 carbon atoms) group, each of which may have a substituent, and
m and p are independently from each other 0 or 1.
The present invention relates to the above-mentioned process for preparing a carbamate compound, wherein the diamine compound is at least one selected from the group consisting of 1,3-bis(aminomethylcyclohexane), 1,4-bis(aminomethylcyclohexane), 2,5-bis(aminomethyl)bicyclo[2.2.1]heptane, 2,6-bis(aminomethyl)bicyclo[2.2.1]heptane, 1,3-bis(aminomethyl)benzene, and 1,4-bis(aminomethyl)benzene.
The present invention relates to the above-mentioned process for preparing a carbamate compound, wherein the amine compound is a diamine compound represented by the following formula (4a):

H₂NH₂C—Z—CH₂NH₂ (4a)

wherein Z is a linear or branched alkylene group which has a main chain having 6 or more carbon atoms and may have a substituent.
The present invention relates to the above-mentioned process for preparing a carbamate compound, wherein Z is a linear or branched alkylene group having a main chain having 6 to 18 carbon atoms, and having 6 to 22 carbon atoms in total.
The present invention relates to the above-mentioned process for preparing a carbamate compound, wherein the diamine compound represented by the formula (4a) is at least one diamine compound selected from the group consisting of 1,8-octanediamine, 1,9-nonanediamine, 1,10-decanediamine, and 1,12-dodecanediamine.
The present invention relates to the above-mentioned process for preparing a carbamate compound, wherein the reaction temperature is 55 to 90°C.

### EFFECT OF THE INVENTION

According to the present invention, a carbamate compound can be prepared from an amine compound and a carbonate compound with high yield and with high selectivity, by an easy and commercially advantageous process under mild conditions.

Further, in the process for preparing a carbamate compound of the present invention, a hydrolase is used, and therefore mixing of an impurity, such as a metal salt or a halide, into a product, which could occur in a conventional process for preparing a carbamate compound, is very unlikely to occur in the method of the present invention, making it possible to provide a chemically safer product.

### MODE FOR CARRYING OUT THE INVENTION

In the invention, a carbamate compound is obtained by reacting an amine compound which has at least one amino group per molecule wherein the amine compound is selected from the group consisting of an aliphatic amine which may be substituted by an alicyclic group or an aromatic group or which may be interrupted by an alicyclic group or an aromatic group, and an alicyclic amine which may be substituted by an aliphatic group, with a carbonate compound in the presence of at least one organic solvent selected from the group consisting of a saturated cyclic hydrocarbon, an unsaturated cyclic hydrocarbon, and a non-cyclic ether by using a hydrolase.

The organic compound having at least one amino group per molecule (hereinafter, referred to as "amine compound"), which is a raw material in the present invention, is an amino group-containing organic compound which has at least one primary amino group (NH₂ group), wherein the organic compound having at least one amino group per molecule is selected from the group consisting of an aliphatic amine optionally substituted by an alicyclic group or an aromatic group or optionally interrupted by an alicyclic group or an aromatic group, and an alicyclic amine optionally substituted by an aliphatic group.

In the present invention, the aliphatic amine is a compound having at least one primary amino group directly bonded to the carbon atom of an aliphatic hydrocarbon group, wherein the aliphatic hydrocarbon group may be substituted by an alicyclic group or an aromatic group, and may be interrupted by an alicyclic group or an aromatic group.
The aliphatic hydrocarbon group is a linear or branched hydrocarbon group which may have an unsaturated bond, and examples of the aliphatic hydrocarbon group include alkyl groups having 1 to 20 carbon atoms, alkenyl groups having 2 to 20 carbon atoms, and alkynyl groups having 2 to 20 carbon atoms.

The aliphatic amine substituted by an alicyclic group is a compound corresponding to the above-defined aliphatic amine in which the hydrocarbon group is substituted by an alicyclic group. The alicyclic group is a hydrocarbon group containing a cyclic structure, which is a saturated or unsaturated, monocyclic or polycyclic (e.g., bi- to tetracyclic) hydrocarbon group having 3 to 20 carbon atoms in total, and examples of alicyclic groups include cycloalkyl groups having 3 to 20 carbon atoms and cycloalkenyl groups having 3 to 20 carbon atoms. Therefore, examples of aliphatic hydrocarbon groups substituted by an alicyclic group include alkyl groups having 1 to 20 carbon atoms substituted by a cycloalkyl group having 3 to 20 carbon atoms.

The aliphatic amine substituted by an aromatic group is a compound corresponding to the above-defined aliphatic amine in which the hydrocarbon group is substituted by an aromatic group, and is also called an araliphatic compound. The aromatic group is a monocyclic or polycyclic hydrocarbon group having 6 to 20 carbon atoms and having an aromatic ring structure, such as a benzene ring or a naphthalene ring, and examples include aryl groups having 6 to 20 carbon atoms. Therefore, examples of aliphatic hydrocarbon groups substituted by an aromatic group include alkyl groups having 1 to 20 carbon atoms substituted by an aryl group having 6 to 20 carbon atoms.

The aliphatic amine interrupted by an alicyclic group is a compound corresponding to the above-defined aliphatic amine in which the carbon-carbon bond of the hydrocarbon group is interrupted by a divalent alicyclic group. Examples of the divalent alicyclic groups include divalent groups obtained by removing a hydrogen atom from the above-defined alicyclic group, such as cycloalkylene groups having 3 to 20 carbon atoms and cycloalkenylene groups having 3 to 20 carbon atoms. Therefore, examples of aliphatic groups interrupted by an alicyclic group include (alkyl having 1 to 15 carbon atoms)-(cycloalkylene having 3 to 20 carbon atoms)-(alkylene having 1 to 15 carbon atoms) groups.

The aliphatic amine interrupted by an aromatic group is a compound corresponding to the above-defined aliphatic amine in which the carbon-carbon bond of the hydrocarbon group is interrupted by a divalent aromatic group. Examples of the divalent aromatic groups include divalent groups obtained by removing a hydrogen atom from the above-defined aromatic group, such as arylene groups having 6 to 20 carbon atoms. Examples of aliphatic groups interrupted by an aromatic group include (alkyl having 1 to 15 carbon atoms)-(arylene having 6 to 20 carbon atoms)-(alkylene having 1 to 15 carbon atoms) groups.

In the present invention, the alicyclic amine is a compound having at least one primary amino group directly bonded to the carbon atom on the ring of an alicyclic group which is monocyclic or polycyclic, and the alicyclic group may be substituted by an aliphatic group. Examples of the alicyclic groups in the alicyclic amine include groups similar to the above-defined alicyclic groups. The alicyclic amine substituted by an aliphatic group is a compound corresponding to the above-defined alicyclic amine in which the alicyclic group is substituted by an aliphatic hydrocarbon group. Examples of the aliphatic hydrocarbon groups include the above-defined aliphatic hydrocarbon groups. Examples of the alicyclic amines substituted by an aliphatic group include cycloalkyl groups having 3 to 20 carbon atoms and being substituted by an alkyl group having 1 to 20 carbon atoms.
In the present invention, an alicyclic amine which is substituted by an aliphatic group and which has a primary amino group directly bonded to the carbon atom of an aliphatic group is involved in the alicyclic amine.

The amine compound may contain in the molecular skeleton thereof a stable bond, such as an ether bond or a thioether bond, and may be substituted by a stable substituent, such as a halogen atom, an alkoxy group, a dialkylamino group, a cyano group, a nitro group, an acetyl group, or an amino group directly bonded to the carbon atom on an aromatic ring.

In the present invention, it is preferred that the amine compound is a compound having a primary amino group or two primary amino groups. Examples of amine compounds having a primary amino group or two primary amino groups include a monoamine compound having a primary amino group in the molecule thereof (hereinafter, referred to as "monoamine compound") represented by the following formula (1):

R¹—(CH₂)ₙ—NH₂ (1)

wherein R¹ and n are as defined above
and a diamine compound having two amino groups in the molecule thereof (hereinafter, referred to as "diamine compound") represented by the following formula (4):

H₂N—(CH₂)ₘ—R3—(CH₂)ₚ—NH₂ (4)

wherein R³, m, and p are as defined above.

Examples of the linear or branched (C₁-C₂₀) alkyl groups having 1 to 20 carbon atoms in R¹ include methyl group, ethyl group, n-propyl group, n-butyl group, n-pentyl group, n-hexyl group, n-heptyl group, n-octyl group, n-nonyl group, n-decyl group, n-undecyl group, n-dodecyl group, isopropyl group, isobutyl group, and t-butyl group. Preferred are linear or branched (C₁-C₁₂) alkyl groups having 1 to 12 carbon atoms, and more preferred are methyl group, ethyl group, n-propyl group, n-butyl group, n-hexyl group, n-dodecyl group, isopropyl group, and t-butyl group.

Examples of the linear or branched (C₂-C₂₀) alkenyl groups having 2 to 20 carbon atoms in R¹ include allyl group, 1-propenyl group, 1-butenyl group, 1-pentenyl group, and isopropanyl group. Preferred are linear or branched (C₂-C₁₂) alkenyl groups having 2 to 12 carbon atoms, and allyl group is more preferred.

Examples of the linear or branched (C₂-C₂₀) alkynyl groups having 2 to 20 carbon atoms in R¹ include ethynyl group, propargyl group, butynyl group, and 1-methyl-2-propynyl group. Preferred are linear or branched (C₂-C₁₂) alkynyl groups having 2 to 12 carbon atoms, and more preferred are an ethynyl group and propargyl group.

The (C₃-C₂₀) cycloalkyl group having 3 to 20 carbon atoms in R¹ is an alicyclic hydrocarbon group which is monocyclic or polycyclic and may be substituted by a linear alkyl group having 1 to 4 carbon atoms, and examples include cyclopropyl group, cyclobutyl group, cyclopentyl group, cyclohexyl group, bicyclo[2.2.1]heptyl group, methylcyclohexyl group, dimethylcyclohexyl group, and an ethylcyclohexyl group. Preferred are (C₃-C₁₂) cycloalkyl groups having 3 to 12 carbon atoms, and more preferred are cyclohexyl group and bicyclo[2.2.1]heptyl group. Examples of the linear alkyl groups having 1 to 4 carbon atoms include methyl group, ethyl group, n-propyl group, and n-butyl group.

The (C₄-C₂₄) cycloalkylalkyl group having 4 to 24 carbon atoms in R¹ is a linear alkyl group having 1 to 4 carbon atoms and being substituted by the above-defined cycloalkyl group having 3 to 20 carbon atoms, and examples include a cyclohexylmethyl group, a cyclohexylethyl group, a trimethylcyclohexylmethyl group, and a norbornylmethyl group. Preferred are cycloalkylalkyl groups having 4 to 14 carbon atoms, that is linear alkyl groups having 1 to 4 carbon atoms which is substituted by cycloalkyl group having 3 to 10 carbon atoms, and cyclohexylmethyl group is more preferred.

Examples of the (C₇-C₂₁) aralkyl groups having 7 to 21 carbon atoms in R¹ include alkyl groups substituted by an aryl group having 6 to 20 carbon atoms. The aryl group having 6 to 20 carbon atoms is a group having a monocyclic or polycyclic aromatic ring structure, and examples include phenyl group, naphthyl group, biphenylyl group, and terphenylyl group (e.g., p-terphenyl-4-yl group and m-terphenyl-3-yl group). The number of carbon atoms of the alkyl group is a number obtained by subtracting the number of carbon atoms of the aryl group from the number of carbon atoms of the aralkyl group. Therefore, examples of (C₇-C₂₁) aralkyl groups having 7 to 21 carbon atoms include benzyl group, phenethyl group, naphthylmethyl group, and m-terphenyl-3-ylmethyl group, and preferred are aralkyl groups having 7 to 13 carbon atoms, and benzyl group is more preferred.
The groups mentioned as examples of R¹ include their various isomers.

The groups mentioned above as examples of R¹ may have a further substituent. Examples of the further substituents in R¹ include halogen atoms (fluorine atom, chlorine atom, bromine atom, and iodine atom); alkoxy groups having 1 to 4 carbon atoms, such as methoxy group, ethoxy group, propoxy group, and butoxy group; dialkylamino groups substituted by two alkyl groups having 1 to 6 carbon atoms, such as dimethylamino group, diethylamino group, and dipropylamino group; cyano group, nitro group, acetyl group, and amino group directly bonded to the benzene ring when R¹ is an aralkyl group.

Thus, with respect to R¹, preferred are linear or branched alkyl groups having 1 to 12 carbon atoms which may have a substituent, such as methyl group, ethyl group, n-propyl group, isopropyl group, n-butyl group, t-butyl group, n-hexyl group, n-dodecyl group, fluoromethyl group, difluoromethyl group, trifluoromethyl group, cyanomethyl group, nitromethyl group, fluoroethyl group, trifluoroethyl group, trichloroethyl group, cyanoethyl group, nitroethyl group, methoxyethyl group, ethoxyethyl group, and t-butoxyethyl group, cycloalkyl groups having 3 to 12 carbon atoms, cycloalkylalkyl groups having 4 to 14 carbon atoms, and aralkyl groups having 7 to 13 carbon atoms which may have a substituent, such as benzyl group, fluorobenzyl group, chlorobenzyl group, bromobenzyl group, iodobenzyl group, methoxybenzyl group, dimethoxybenzyl group, nitrobenzyl group, dinitrobenzyl group, cyanobenzyl group, and aminobenzyl group; and especially preferred are methyl group, ethyl group, n-propyl group, isopropyl group, n-butyl group, t-butyl group, n-hexyl group, n-dodecyl group, cyclohexyl group, cyclohexylmethyl group, and benzyl group.

Therefore, with respect to the monoamine compound represented by the formula (1), especially preferred are n-hexylamine, n-dodecylamine, cyclohexylmethylamine, and benzylamine.

Examples of the linear or branched alkylene groups having 1 to 30 carbon atoms in R³ include linear alkylene groups, such as methylene group, ethylene group, n-propylene group, n-butylene group, n-pentylene(pentamethylene) group, n-hexylene(hexamethylene) group, n-heptylene(heptamethylene) group, n-octylene(octamethylene) group, n-nonylene(nonamethylene) group, n-decylene(decamethylene) group, and n-dodecylene(dodecamethylene) group, and branched alkylene groups, such as 2-methylpropylene group, 2-methylhexylene group, and tetramethylethylene group. Preferred are linear or branched alkylene groups having 1 to 20 carbon atoms, and more preferred are linear alkylene groups having 1 to 20 carbon atoms, and further preferred are methylene group, ethylene group, propylene group, butylene group, pentylene group, hexylene group, heptylene group, octylene group, nonylene group, decylene group, and dodecylene group.

The cycloalkylene group having 3 to 20 carbon atoms in R³ is a monocyclic or polycyclic hydrocarbon group which may be substituted by a linear alkyl group having 1 to 4 carbon atoms, and examples include cyclopropylene group, cyclobutylene group, cyclopentylene group, cyclohexylene group, and bicyclo[2.2.1]heptane-2,6-diyl group. Preferred are cycloalkylene groups having 3 to 12 carbon atoms, and more preferred are cyclohexylene group and bicyclo[2.2.1]heptane-2,6-diyl group.

Examples of the linear alkylene groups having 1 to 4 carbon atoms in the (linear alkylene having 1 to 4 carbon atoms)-(cycloalkylene having 3 to 20 carbon atoms)-(linear alkylene having 1 to 4 carbon atoms) group in R³ include methylene group, ethylene group, propylene group, and butylene group. Examples of the (linear alkylene having 1 to 4 carbon atoms)-(cycloalkylene having 3 to 20 carbon atoms)-(linear alkylene having 1 to 4 carbon atoms) groups include methylene-cyclopentylene-methylene group, ethylene-cyclopentylene-ethylene group, and methylene-cyclohexylene-methylene group. Preferred are (linear alkylene having 1 to 4 carbon atoms)-(cyclohexylene having 3 to 12 carbon atoms)-(linear alkylene having 1 to 4 carbon atoms) groups, and methylene-cyclohexylene-methylene group is more preferred.

With respect to the (linear alkylene having 1 to 4 carbon atoms)-(cycloalkylene having 3 to 20 carbon atoms) group in R³, preferred are cycloalkylene groups having 3 to 12 carbon atoms which is substituted by (linear alkylene having 1 to 4 carbon atoms)-(linear alkyl having 1 to 4 carbon atoms) group, and methylene-trimethylcyclohexylene group is more preferred.

With respect to the (linear alkylene having 1 to 4 carbon atoms)-(arylene having 6 to 20 carbon atoms)-(linear alkylene having 1 to 4 carbon atoms) group in R³, preferred are (linear alkylene having 1 to 4 carbon atoms)-phenylene-(linear alkylene having 1 to 4 carbon atoms) groups, and xylylene group is more preferred.
These groups include their various isomers.

The hydrocarbon group in R³ may have a substituent. Examples of the substituents in R³ include groups similar to the substituents for the hydrocarbon group in R¹. When R³ is (linear alkylene having 1 to 4 carbon atoms)-(arylene having 6 to 20 carbon atoms)-(linear alkylene having 1 to 4 carbon atoms) group, an example of the substituent in R³ include a primary amino group directly bonded to the aromatic carbon atom of the arylene group.

Thus, with respect to R³, preferred are linear or branched alkylene groups having 1 to 30 carbon atoms, (linear alkylene having 1 to 4 carbon atoms)-(cycloalkylene having 3 to 20 carbon atoms)-(linear alkylene having 1 to 4 carbon atoms) groups, (linear alkylene having 1 to 4 carbon atoms)-(arylene having 6 to 20 carbon atoms)-(linear alkylene having 1 to 4 carbon atoms) groups, cycloalkylene groups having 3 to 20 carbon atoms, and (linear alkylene having 1 to 4 carbon atoms)-(cycloalkylene having 3 to 20 carbon atoms) groups; more preferred are linear or branched alkylene groups having 1 to 30 carbon atoms, (linear alkylene having 1 to 4 carbon atoms)-(cycloalkylene having 3 to 12 carbon atoms)-(linear alkylene having 1 to 4 carbon atoms) groups, (linear alkylene having 1 to 4 carbon atoms)-phenylene-(linear alkylene having 1 to 4 carbon atoms) groups, cycloalkylene groups having 3 to 12 carbon atoms, and (linear alkylene having 1 to 4 carbon atoms)-(cycloalkylene groups having 3 to 12 carbon atoms which is substituted by linear alkyl having 1 to 4 carbon atoms) group; and especially preferred are methylene group, ethylene group, propylene group, butylene group, pentamethylene group, hexamethylene group, heptamethylene group, octamethylene group, nonamethylene group, decamethylene group, dodecamethylene group, cyclohexylene group, methylene-trimethylcyclohexylene group, cyclohexylenedimethylene group, and xylylene group.

In the present invention, with respect to the substrate specificity of the enzyme catalyst to the linear diamine compound, especially preferred is the compound wherein each of m and p is 1 and R³ is a linear or branched alkylene group having a main chain having 6 or more carbon atoms because the reactivity with the carbonate compound is remarkably improved. Therefore, in the present invention, the amine compound is especially preferably a diamine compound represented by the following formula (4a):

H₂NH₂C—Z—CH₂NH₂ (4a)

wherein Z is as defined above.

The diamine compound represented by the formula (4a) is an amino group-containing organic compound having two aminomethyl groups (NH₂CH₂ groups) in the same molecule wherein the aminomethyl groups are bonded to the both ends of a linear or branched alkylene group having a main chain having 6 or more carbon atoms. The diamine compound may be used individually or a mixture of a plurality of the diamine compounds may be used. In the present invention, the main chain indicates the shortest carbon chain connecting two aminomethyl groups.

In the present invention, Z is a linear or branched alkylene group having a main chain having 6 or more carbon atoms. Examples of the linear or branched alkylene groups having a main chain having 6 or more carbon atoms include linear alkylene groups having 6 or more carbon atoms and being unsubstituted or substituted by at least one linear or branched alkyl group, such as linear alkylene groups having 6 or more carbon atoms whichi is unsubstituted or substituted by a linear or branched alkyl group having 1 to 16 carbon atoms.

Examples of the linear alkylene groups having 6 or more carbon atoms include hexamethylene group, heptamethylene group, an octamethylene group, nonamethylene group, decamethylene group, undecamethylene group, dodecamethylene group, tridecamethylene group, tetradecamethylene group, pentadecamethylene group, hexadecamethylene group, heptadecamethylene group, octadecamethylene group, nonadecamethylene group, and eicosamethylene group.

Examples of the linear or branched alkyl groups having 1 to 16 carbon atoms include methyl group, ethyl group, n-propyl group, n-butyl group, n-pentyl group, n-hexyl group, n-heptyl group, n-octyl group, n-nonyl group, n-decyl group, n-undecyl group, n-dodecyl group, isopropyl group, isobutyl group, and t-butyl group.

In the present invention, from the viewpoint of obtaining a dicarbamate compound with high yield and in an even shorter reaction time using a reduced amount of an enzyme, the number of carbon atoms of the main chain is preferably 6 to 18, more preferably 6 to 12, further preferably 7 to 10.

Thus, with respect to Z in the diamine compound represented by the formula (4a), preferred are linear or branched alkylene groups having a main chain having 6 to 18 carbon atoms, and having 6 to 22 carbon atoms in total, more preferred are linear or branched alkylene groups having a main chain having 6 to 12 carbon atoms, and having 6 to 16 carbon atoms in total, further preferred are linear or branched alkylene groups having a main chain having 7 to 10 carbon atoms, and having 7 to 14 carbon atoms in total, and especially preferred are octamethylene group and decamethylene group.

In the present invention, Z may have a substituent. Examples of the substituents in Z include groups similar to the substituents for the hydrocarbon group in R¹.

The diamine compound represented by the formula (4a) is especially preferably at least one diamine compound selected from the group consisting of 1,8-octanediamine, 1,9-nonanediamine, 1,10-decanediamine, and 1,12-dodecanediamine.

With respect to the amine compound which is a raw material in the present invention, preferred is a diamine compound from which a biscarbamate compound used as a raw material for diisocyanate is obtained. In the present invention, it is more preferred that the diamine compound is at least one selected from the group consisting of 1,6-hexanediamine, 1,8-octanediamine, 1,9-nonanediamine, 1,10-decanediamine, 1,12-dodecanediamine, isophoronediamine, 1,3-bis(aminomethylcyclohexane), 1,4-bis(aminomethylcyclohexane), 4,4'-methylenebis(cyclohexaneamine), 2,5-bis(aminomethyl)bicyclo[2,2,1]heptane, 2,6-bis(aminomethyl)bicyclo[2,2,1]heptane, 1,3-bis(aminomethyl)benzene, and 1,4-bis(aminomethyl)benzene.

In the present invention, when the amine compound is a monoamine compound, the process for preparing a carbamate compound of the present invention is represented by the reaction formula [I] shown below. In reaction formula [I], a monocarbamate compound represented by the formula (3) is obtained by reacting a monoamine compound represented by the formula (1) with a carbonate compound represented by the formula (2) in at least one organic solvent selected from the group consisting of a saturated cyclic hydrocarbon, an unsaturated cyclic hydrocarbon, and a non-cyclic ether by using a hydrolase.

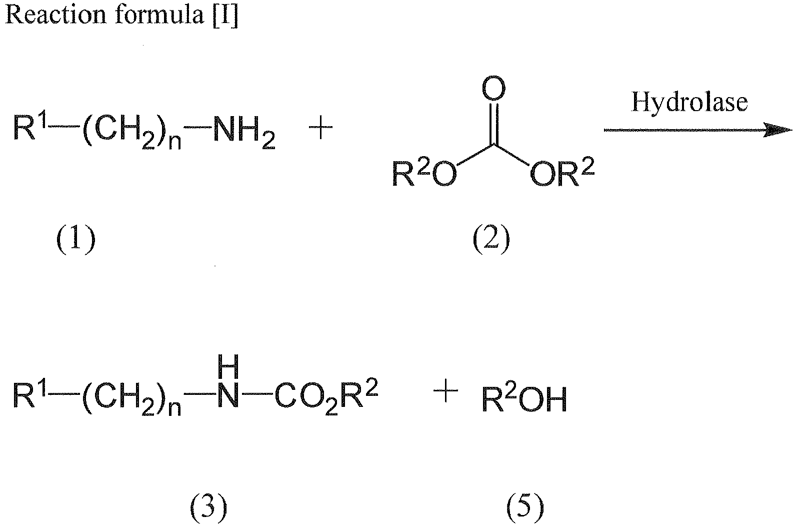

Wherein R¹, R², and n are as defined above.

In the formula (2), examples of the monovalent hydrocarbon groups which may have a substituent in R² include groups having the same meanings as those of R¹ defined in the formula (1). With respect to the hydrocarbon group in R², preferred are linear or branched alkyl groups having 1 to 20 carbon atoms, preferably having 1 to 6 carbon atoms, such as methyl group, ethyl group, n-propyl group, i-propyl group, and n-butyl group, and especially preferred are methyl group and ethyl group.

The hydrocarbon group in R² may have a substituent. Examples of the substituents for the hydrocarbon group in R² include halogen atoms (fluorine atom, chlorine atom, bromine atom, and iodine atom); alkoxy groups having 1 to 4 carbon atoms, such as methoxy group, ethoxy group, propoxy group, and butoxy group; dialkylamino groups substituted by two alkyl groups having 1 to 4 carbon atoms, such as dimethylamino group, diethylamino group, and dipropylamino group; cyano group; and nitro group.

Thus, with respect to the carbonate compound represented by the formula (2), dimethyl carbonate or diethyl carbonate is preferred.

### [Reaction formula II]

In the present invention, when the amine compound is a diamine compound, the process for preparing a carbamate compound of the present invention is represented by the reaction formula [II] shown below. In reaction formula [II], a biscarbamate compound represented by the formula (6) is obtained by reacting a diamine compound represented by the formula (4) with a carbonate compound represented by the formula (2) in at least one organic solvent selected from the group consisting of a saturated cyclic hydrocarbon, an unsaturated cyclic hydrocarbon, and a non-cyclic ether by using a hydrolase.

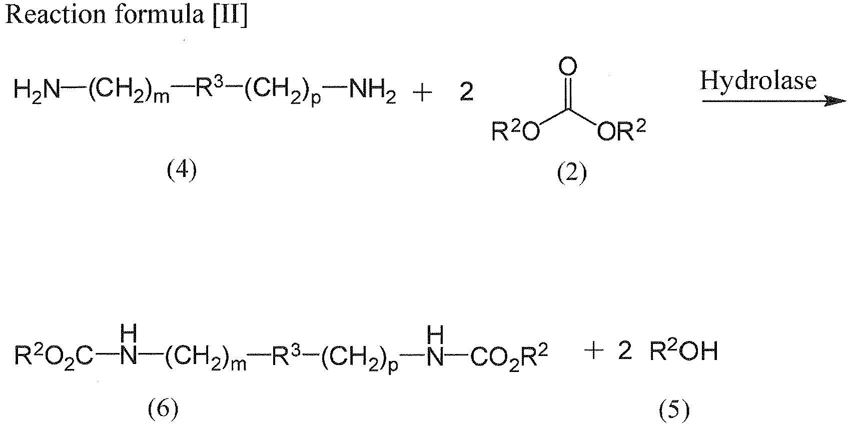

Wherein R², R³, m, and p are as defined above.

Further, in the present invention, when the diamine compound is a diamine compound represented by the formula (4a), the process for preparing a carbamate compound is represented by the reaction formula [IIa] shown below. In reaction formula [IIa], a dicarbamate compound represented by the formula (6a) is obtained by reacting a diamine compound represented by the formula (4a) with a carbonate compound represented by the formula (2) by using a hydrolase.

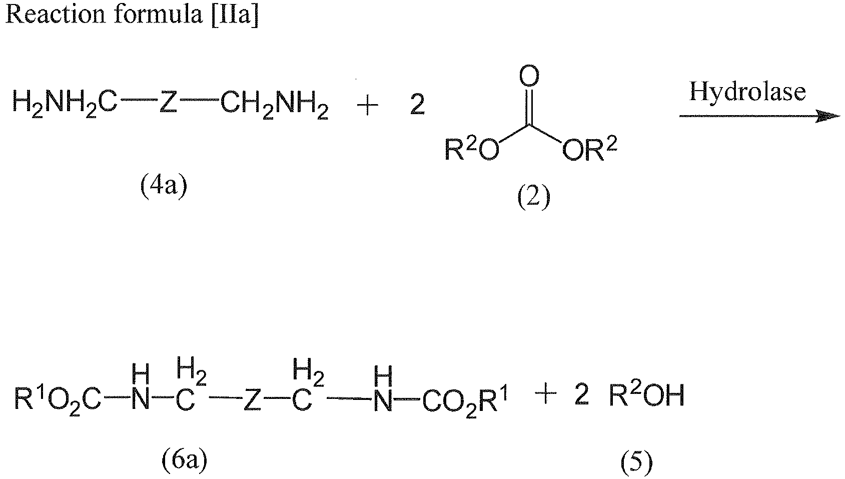

Wherein Z and R² are as defined above.

In reaction formula [I], the amount of the carbonate compound represented by the formula (2) is 1 to 100 mol based on 1 mol of the monoamine compound represented by the formula (1), preferably 1 to 50 mol, more preferably 2 to 20 mol, further preferably 2 to 15 mol, especially preferably 2 to 10 mol.
In reaction formulae [II] and [IIa], the amount of the carbonate compound represented by the formula (2) is 2 to 200 mol based on 1 mol of the diamine compound represented by the formula (4) or formula (4a), preferably 2 to 100 mol, more preferably 4 to 40 mol, further preferably 4 to 30 mol, especially preferably 4 to 20 mol.

Examples of the hydrolases used in the present invention include a protease, an esterase, a lipase, etc., but preferred are esterase from pig liver (PLE), lipase from pig liver (PPL), and lipase of microorganisms which can be isolated from yeasts or bacteria; further preferred are lipase originated from *Burkholderia cepacia* (*Pseudomonas cepacia)* {e.g., Amano PS (manufactured by Amano Enzyme Inc.)}, lipase originated from *Candida antarctica* {e.g., Novozym 435 (manufactured by Novozymes)}, lipase originated from *Rhizomucor Miehei* {e.g., Lipozyme RM IM (manufactured by Novozymes)}, lipase originated from *Thermomyces lanuginosus* (Lipase TL), and lipase originated from *Mucor Miehei* (Lipase MM); and lipase originated from *Candida antarctica* is especially preferably used. With respect to these hydrolases, commercially available products in a natural form or in an immobilized enzyme form can be used as such, and may be used individually or in combination.

With respect to the above hydrolase, there can be used the commercially available hydrolase in a natural form or in an immobilized enzyme form, which has been subjected to chemical treatment or physical treatment. In the present invention, the hydrolase is preferably immobilized on a support, more preferably a hydrolase immobilized on a support which is incorporated as a fixed bed to the inner side of a reaction vessel.

An example of the above-mentioned chemical treatment or physical treatment method includes a method in which a hydrolase is dissolved in a buffer (in an organic solvent may present if necessary) and the resultant solution is directly or stirred and then subjected to freeze-drying. The freeze-drying in the present invention is a method, for example, described in J. Am. Chem. Soc., 122(8), 1565-1571 (2000), in which an aqueous solution of a substance or a substance containing moisture is quickly frozen at a temperature of ice point or lower, and the pressure of the container containing the frozen substance is reduced to the water vapor pressure of the frozen substance or less so that water undergoes sublimation, removing water and thus drying the substance. The above treatment can improve the catalytic activity (e.g., reactivity or selectivity).

In reaction formula [I], the amount of the hydrolase used is preferably 0.1 to 1,000 mg based on 1 g of the monoamine compound represented by the formula (1), more preferably 1 to 200 mg, especially preferably 10 to 100 mg.
In reaction formula [II] or reaction formula [IIa], the amount of the hydrolase used is preferably 0.1 to 1,000 mg based on 1 g of the diamine compound represented by the formula (4) or formula (4a), more preferably 1 to 200 mg, especially preferably 10 to 100 mg.

The reaction in the present invention is conducted by reacting a monoamine compound represented by the formula (1) or a diamine compound represented by the formula (4) or formula (4a) with a carbonate compound represented by the formula (2) in at least one organic solvent selected from the group consisting of a saturated cyclic hydrocarbon, an unsaturated cyclic hydrocarbon, and a non-cyclic ether. By using the above organic solvent, a carbamate compound can be obtained with high yield.

With respect to the at least one organic solvent selected from the group consisting of a saturated cyclic hydrocarbon, an unsaturated cyclic hydrocarbon, and a non-cyclic ether used in the reaction in the present invention, there is no particular limitation as long as the organic solvent does not deactivate the enzyme.

Examples of saturated cyclic hydrocarbons include unsubstituted cycloalkane solvents having 5 to 10 carbon atoms, such as cyclopentane, cyclohexane, cycloheptane, and isopropylcyclohexane; and cycloalkane solvents having 5 to 10 carbon atoms and being substituted by halogen, such as chlorocyclopentane and chlorocyclohexane. Preferred are unsubstituted cycloalkane solvents having 5 to 10 carbon atoms, and cyclohexane is more preferred.
Examples of unsaturated cyclic hydrocarbons include aromatic hydrocarbons, such as benzene, toluene, xylene, and mesitylene; and cycloalkene solvents having 5 to 10 carbon atoms, such as cyclopentene and cyclohexene. Preferred are aromatic hydrocarbons, and more preferred are toluene and xylene.
Examples of non-cyclic ethers include aliphatic ethers, e.g., dialkyl ethers having 2 to 8 carbon atoms, such as diethyl ether, t-butyl methyl ether, and diisopropyl ether, and cycloalkyl alkyl ethers having 5 to 18 carbon atoms, such as cyclopentyl methyl ether and cyclopentyl ethyl ether; and aromatic ethers having 7 to 18 carbon atoms, such as benzyl phenyl ether, benzyl methyl ether, diphenyl ether, di(p-tolyl) ether, and dibenzyl ether. Preferred are aliphatic ethers, and diisopropyl ether is more preferred.
These organic solvents may be used individually or in combination.

The amount of the above-mentioned at least one organic solvent selected from the group consisting of a saturated cyclic hydrocarbon, an unsaturated cyclic hydrocarbon, and a non-cyclic ether is preferably 2 to 200 mL based on 1 g of the monoamine compound represented by the formula (1) or the diamine compound represented by the formula (4) or formula (4a), more preferably 5 to 50 mL, especially preferably 2 to 20 mL.

The reaction in the present invention is conducted by, for example, a method in which a monoamine compound represented by the formula (1) or a diamine compound represented by the formula (4) or formula (4a), a carbonate compound represented by the formula (2), at least one organic solvent selected from the group consisting of a saturated cyclic hydrocarbon, an unsaturated cyclic hydrocarbon, and a non-cyclic ether, and a hydrolase are mixed together and subjected to reaction while stirring.

With respect to the reaction temperature in the reaction in the present invention, there is no particular limitation as long as the enzyme is not deactivated at that temperature. However, for obtaining a desired carbamate compound with high yield, the reaction temperature is preferably 55 to 90°C, more preferably 60 to 90°C, especially preferably 65 to 90°C. With respect to the reaction pressure, there is no particular limitation, but the reaction is preferably conducted under atmospheric pressure or a reduced pressure.

It is desired that the reaction in the present invention is conducted in a range selected depending on the properties of the enzyme used in the reaction such that the enzyme is not deactivated. With respect to the pH of the reaction solution, there is no particular limitation, but the pH value is preferably 5 to 9, more preferably 6 to 8.5, especially preferably 6.5 to 8.

Further, the present invention is advantageous in that the reaction in the present invention is generally of a homogeneous system and hence the catalyst can be reused and the after-treatment of the reaction is easy. Specifically, after completion of the reaction, the catalyst is removed by filtration, and a product can be obtained by concentrating the resultant filtrate. Alternatively, a product can be obtained by an operation of crystallization from the resultant filtrate.

With respect to the production apparatus used for the reaction in the present invention, there is no particular limitation, and examples include general production apparatuses, such as a reaction vessel and a heating (cooling) apparatus. In the present invention, preferred is an apparatus in which a hydrolase is immobilized on a support which is incorporated as a fixed bed to the inner side of a reaction vessel. Therefore, the reaction in the present invention is preferably a reaction comprising the step of allowing the monoamine compound represented by the formula (1) or the diamine compound represented by the formula (4) or formula (4a) and the carbonate compound represented by the formula (2) to pass through the reaction vessel.

Further, the monocarbamate compound represented by the formula (3) or biscarbamate compound represented by the formula (6) or formula (6a), which is obtained by the method of the present invention, can be further purified by a general method, such as distillation, separation, extraction, crystallization, recrystallization, or column chromatography.

The monocarbamate compound represented by the formula (3) or biscarbamate compound represented by the formula (6) or formula (6a), which is obtained by the process of the present invention, is produced using a hydrolase. Therefore, mixing of an impurity, such as a metal salt or a halide, into a product, which could occur in a conventional process for preparing a carbamate compound, is very unlikely to occur in the method of the present invention, making it possible to obtain a chemically safer product.

### EXAMPLES

Next, the present invention is explained more specifically by referring to Examples, but the scope of the present invention is not limited by these.

With respect to the obtained desired product, the confirmation of structure was carried out by IR, NMR spectrum analyses, etc. In addition, the measurement of purity was carried out by using high performance liquid chromatography.

### Example 1 (Synthesis of n-hexylmonomethyl carbamate)

In a vessel made of glass having an internal volume of about 19 ml and being equipped with a stirring device, a thermometer and a condenser were charged 500 mg (4.94 mmol) of n-hexylamine, 2.67 g (29.65 mmol) of dimethyl carbonate, 5 mL of toluene, and 25 mg of lipase from *Candida Antarctica* (Novozym 435 (trade name), manufactured by Novozymes) and mixed, and the mixture was reacted under stirring at 70°C for 24 hours. After completion of the reaction, the reaction solution was filtered, and the filtrate was washed with toluene. The filtrate solution was concentrated to obtain 747 mg of n-hexylmonomethyl carbamate in the form of white solids (yield of the isolated product, based on n-hexylamine: 95%).
Physical properties of the obtained n-hexylmonomethyl carbamate were as follows.
EI-MS(m/z); 159 [M].
CI-MS (m/z); 160 [M+1].
¹H-NMR (CDCl₃, δ (ppm)); 0.94 (3H, t, J = 7.32Hz), 1.40 (2H, tq, J = 7.32,Hz), 1.66 (2H, tt, J = 6.59, Hz), 3.78(3H, s), 4.14 (2H, t, J = 6.59Hz).
¹³C-NMR (CDCl₃, δ (ppm)); 14.0, 22.6, 26.4, 30.0, 31.5, 41.2, 52.0, 157.2.
IR (Liquid membrane method, cm⁻¹); 640, 688, 726, 780, 936, 1017, 1043, 1113, 1147, 1193, 1254, 1343, 1379, 1466, 1539, 1704, 2860, 2931, 2957, 3338.
EA; Calcd: C, 60.35%; H, 10.76%; N, 8.80%
Found: C, 59.54%; H, 10.30%; N, 8.63%

### Example 2 (Synthesis of n-dodecylmonomethyl carbamate)

In a vessel made of glass having an internal volume of about 19 ml and being equipped with a stirring device, a thermometer and a condenser were charged 500 mg (2.70 mmol) of n-dodecylamine, 1.46 g (16.19 mmol) of dimethyl carbonate, 5 mL of toluene, and 25 mg of lipase from *Candida Antarctica* (Novozym 435 (trade name), manufactured by Novozymes) and mixed, and the mixture was reacted under stirring at 70°C for 5 hours. After completion of the reaction, the reaction solution was filtered, and the filtrate was washed with toluene. The filtrate solution was concentrated to obtain 637 mg of n-dodecylmonomethyl carbamate in the form of white solids (yield of the isolated product, based on n-dodecylamine: 97%).
Physical properties of the obtained n-dodecylmonomethyl carbamate were as follows.
EI-MS (m/z); 243 [M].
CI-MS (m/z); 244 [M+1].
¹H-NMR (CDCl3, δ (ppm)); 0.94 (3H, t, J = 7.32Hz), 1.40 (2H, tq, J = 7.32, Hz), 1.66 (2H, tt, J = 6.59, Hz), 3.78 (3H, s), 4.14 (2H, t, J = 6.59Hz).
¹³C-NMR (CDCl₃, δ (ppm)); 14.1, 22.7, 26.8, 29.3, 29.4, 29.56, 29.60, 29.64, 29.7, 30.0, 31.9, 41.1, 52.0, 157.1.
IR (KBr method, cm⁻¹); 556, 610, 659, 724, 764, 783, 888, 936, 991, 1005, 1022, 1046, 1130, 1145, 1199, 1229, 1250, 1270, 1294, 1320, 1343, 1372, 1464, 1479, 1533, 1692, 2851, 2922, 2955, 3351.
EA; Calcd: C, 69.09%; H, 12.01%; N, 5.75%.
Found: C, 68.78%; H, 11.65%; N, 5.74%.

### Example 3 (Synthesis of cyclohexylmonomethyl carbamate)

In a vessel made of glass having an internal volume of about 19 ml and being equipped with a stirring device, a thermometer and a condenser were charged 500 mg (5.04 mmol) of cyclohexylamine, 2.73 g (30.25 mmol) of dimethyl carbonate, 5 mL of toluene, and 100 mg of lipase from *Candida Antarctica* (Novozym 435 (trade name), manufactured by Novozymes) and mixed, and the mixture was reacted under stirring at 70°C for 60 hours. After completion of the reaction, the reaction solution was filtered, and the filtrate was washed with toluene. The filtrate solution was concentrated to obtain 769 mg of cyclohexylmonomethyl carbamate in the form of white solids (yield of the isolated product, based on cyclohexylamine: 97%).
Physical properties of the obtained cyclohexylmonomethyl carbamate were as follows.
EI-MS (m/z); 157 [M].
CI-MS (m/z); 158 [M+1].
¹H-NMR (CDCl₃, δ (ppm)); 3.68 (3H, s), 4.35 (2H, d, J = 4.35Hz), 5.11 (1H, s), 7.23 - 7.36 (5H, m).
¹³C-NMR (CDCl₃, δ (ppm)); 45.1, 52.2, 125.8, 127.5, 128.7, 138.6, 157.1.
IR(KBr, em⁻¹); 456, 544, 609, 699, 737, 779, 819, 877, 915, 994, 1029, 1043, 1081, 1143, 1195, 1262, 1342, 1365, 1434, 1455, 1497, 1532, 1605, 1705, 2843, 2950, 2995, 3031,3065,3088,3333.
EA; Calcd: C, 61.12%; H, 9.62%; N, 8.91%
Found: C, 61.09%; H, 9.35%; N, 8.82%.

### Example 4 (Synthesis of cyclohexylmethylmonomethyl carbamate)

In a vessel made of glass having an internal volume of about 19 ml and being equipped with a stirring device, a thermometer and a condenser were charged 500 mg (4.42 mmol) of cyclohexylmethylamine, 2.23 g (26.50 mmol) of dimethyl carbonate, 5 mL of toluene, and 25 mg of lipase from *Candida Antarctica* (Novozym 435 (trade name), manufactured by Novozymes) and mixed, and the mixture reacted under stirring at 70°C for 15 hours. After completion of the reaction, the reaction solution was filtered, and the filtrate was washed with toluene. The filtrate solution was concentrated to obtain 741 mg of cyclohexylmethylmonomethyl carbamate in the form of white solids (yield of the isolated product, based on cyclohexylmethylamine: 98%).
Physical properties of the obtained cyclohexylmethylmonomethyl carbamate were as follows.
EI-MS (m/z); 171 [M].
CI-MS (m/z); 172 [M+1].
¹H-NMR (CDCl₃, δ (ppm)); 0.85 - 1.74 (11H, m), 2.99 - 3.03 (2H, m), 3.66 (3H, m), 4.80 (1H, s).
¹³C-NMR (CDCl₃, δ (ppm)); 25.8, 26.4, 30.7, 38.3, 47.4, 52.0, 157.3.
IR(KBr, cm⁻¹); 455, 536, 570, 655, 708, 784, 845, 892, 941, 973, 1031, 1057, 1081, 1144, 1195, 1225, 1250, 1264, 1281, 1301, 1317, 1341, 1370, 1380, 1445, 1464, 1480, 1546, 1654, 1687, 1707, 2789, 2850, 2895, 2919, 3020, 3066, 3312.
EA; Calcd: C, 63.13%; H, 10.01%; N, 8.18%.
Found: C, 63.08%; H, 9.67%; N, 8.17%.

### Example 5 (Synthesis of benzylmonomethyl carbamate)

In a vessel made of glass having an internal volume of about 19 ml and being equipped with a stirring device, a thermometer and a condenser were charged 500 mg (4.67 mmol) of benzylamine, 2.36 g (28.00 mmol) of dimethyl carbonate, 5 mL of toluene, and 25 mg of lipase from *Candida Antarctica* (Novozym 435 (trade name), manufactured by Novozymes) and mixed, and the mixture was reacted under stirring at 70°C for 15 hours. After completion of the reaction, the reaction solution was filtered, and the filtrate was washed with toluene. The filtrate solution was concentrated to obtain 763 mg of benzylmonomethyl carbamate in the form of a pale yellow liquid (yield of the isolated product, based on benzylamine: 99%).
Physical properties of the obtained benzylmonomethyl carbamate were as follows.
EI-MS (m/z); 165 [M].
CI-MS (m/z); 166 [M+1].
¹H-NMR (CDCl₃, δ (ppm)); 0.94 (3H, t, J = 7.32Hz), 1.40 (2H, tq, J = 7.32, Hz), 1.66 (2H, tt, J = 6.59, Hz), 3.78(3H,s), 4.14 (2H, t, J = 6.59Hz).
¹³C-NMR (CDCl₃, δ (ppm)); 13.7, 18.9, 30.7, 54.6, 68.0, 155.9.
IR (Liquid membrane method, cm⁻¹); 794, 957, 1013, 1103, 1168, 1200, 1269, 1363, 1388, 1443, 1747, 2866, 2956.
EA; Calcd: C, 65.44%; H, 6.71%; N, 8.48%
Found: C, 64.41%; H, 6.44%; N, 8.17%.

### Example 6 (Synthesis of 1,6-hexamethylenedimethyl carbamate)

In a vessel made of glass having an internal volume of about 19 ml and being equipped with a stirring device, a thermometer and a condenser were charged 2.32 g (19.97 mmol) of 1,6-hexamethylenediamine, 10.81 g (119.79 mmol) of dimethyl carbonate, 46.0 g of toluene, and 120 mg of lipase from *Candida Antarctica* (Novozym 435 (trade name), manufactured by Novozymes) and mixed, and the mixture was reacted under stirring at 70°C for 88 hours. After completion of the reaction, the reaction solution was filtered, and the filtrate was washed with toluene. The filtrate solution was concentrated to obtain 4.45 g of 1,6-hexamethylenedimethyl carbamate in the form of white solids (yield of the isolated product, based on 1,6-hexamethylenediamine: 96%).
Physical properties of the obtained 1,6-hexamethylenedimethyl carbamate were as follows.
EI-MS (m/z); 232 [M].
CI-MS (m/z); 233 [M+1].
¹H-NMR (CDCl₃,δ (ppm)); 0.94 (3H, t, J = 7.32Hz), 1.40 (2H, tq, J = 7.32, Hz), 1.66 (2H, tt, J = 6.59, Hz), 3.78 (3H,s), 4.14 (2H, t, J = 6.59Hz).
¹³C-NMR (CDCl₃, δ(pom)); 26.2, 29.9, 40.8, 52.0, 157.1.
IR (KBr, em⁻¹); 424, 566, 627, 704, 735, 781, 804, 934, 1009, 1053, 1141, 1194, 1224, 1265, 1341, 1436, 1480, 1538, 1691, 2777, 2871, 2914, 2944, 3047, 3339.
EA; Calcd: C, 51.71%; H, 8.68%; N, 12.06%
Found: C, 51.77%; H, 8.27%; N, 12.02%.

### Example 7 (Synthesis of 1,9-nonamethylenedimethyl carbamate)

In a vessel made of glass having an internal volume of about 19 ml and being equipped with a stirring device, a thermometer and a condenser were charged 250 mg (1.58 mmol) of 1,9-nonanediamine, 854 mg (9.48 mmol) of dimethyl carbonate, 2.5 mL of toluene, and 13 mg of lipase from *Candida Antarctica* (Novozym 435 (trade name), manufactured by Novozymes) and mixed, and the mixture was reacted under stirring at 70°C for 48 hours. After completion of the reaction, the reaction solution was filtered, and the filtrate was washed with toluene. The filtrate solution was concentrated to obtain 425 mg of 1,9-nonamethylenedimethyl carbamate in the form of white solids (yield of the isolated product, based on 1,9-nonanediamine: 98%).
Physical properties of the obtained 1,9-nonamethylenedimethyl carbamate were as follows.
EI-MS (m/z); 274 [M].
CI-MS (m/z); 275 [M+1].
¹H-NMR (CDCl₃, δ (ppm)); 1.29 (10H, s(br)), 1.48 (4H, tq, J = 6.59Hz), 3.16 (4H, tt, J = 6.59Hz), 3.66 (6H, s), 4.73 (2H, s).
¹³C-NMR (CDCl₃, δ (ppm)); 26.7, 29.1, 29.4, 30.0, 41.1, 52.0,157.1.
IR(KBr method, cm-¹); 464, 563, 612, 668, 705, 724, 746, 784, 881, 931, 980, 1021, 1056, 1142, 1201, 1254, 1288, 1328, 1352, 1370, 1479, 1531, 1693, 2852, 2921, 3348.
EA; Calcd: C, 56.91%; H, 9.55%; N, 10.21%
Found: C, 57.17%; H, 9.45%; N, 10.18%.

### Example 8 (Synthesis of 1,12-dodecamethylenedimethyl carbamate)

In a vessel made of glass having an internal volume of about 19 ml and being equipped with a stirring device, a thermometer and a condenser were charged 500 mg (2.50 mmol) of 1,12-dodecanediamine, 1.35 g (14.97 mmol) of dimethyl carbonate, 5 mL of toluene, and 25 mg of lipase from *Candida Antarctica* (Novozym 435 (trade name), manufactured by Novozymes) and mixed, and the mixture was reacted under stirring at 70°C for 19 hours. After completion of the reaction, the reaction solution was filtered, and the filtrate was washed with toluene. The filtrate solution was concentrated to obtain 774 mg of 1,12-dodecamethylenedimethyl carbamate in the form of white solids (yield of the isolated product, based on 1,12-dodecanediamine: 98%).
Physical properties of the obtained 1,12-dodecamethylenedimethyl carbamate were as follows.
EI-MS (m/z); 316 [M].
CI-MS (m/z); 317[M+1].
¹H-NMR(CDCl₃, δ (ppm)); 1.26 - 1.29 (16H,m), 1.48 (4H, tq, J = 6.59Hz), 3.16 (4H, tt, J = 6.59Hz), 3.66 (6H, s), 4.71(2H,s).
¹³C-NMR (CDCl₃, δ(ppm)); 26.7, 29.3, 29.5, 30.0, 41.1, 52.0, 157.1.
IR (KBr method, cm⁻¹);442, 485, 559, 619, 708, 724, 767, 785, 933, 983, 1011, 1033, 1057, 1097, 1143, 1197, 1244, 1273, 1317, 1355, 1435, 1466, 1479, 1532, 1689, 2852, 2872, 2923, 2941, 3044, 3346.
EA; Calcd: C, 60.73%; H, 10.19%; N, 8.85%
Found: C, 60.43%; H, 9.90%; N, 8.81%.

### Example 9 (Synthesis of 1,3-bis(methoxycarbonylaminomethyl)cyclohexane)

In a vessel made of glass having an internal volume of about 19 ml and being equipped with a stirring device, a thermometer and a condenser were charged 500 mg (3.52 mmol) of 1,3-bis(aminomethyl)cyclohexane, 1.90 g (21.09 mmol) of dimethyl carbonate, 5 mL of toluene, and 25 mg of lipase from *Candida Antarctica* (Novozym 435 (trade name), manufactured by Novozymes) and mixed, and the mixture was reacted under stirring at 70°C for 42 hours. After completion of the reaction, the reaction solution was filtered, and the filtrate was washed with toluene. The filtrate solution was concentrated to obtain 872 mg of 1,3-bis(methoxycarbonylaminomethyl) cyclohexane in the form of white solids (yield of the isolated product, based on 1,3-bis(aminomethyl)cyclohexane: 96%).
Physical properties of the obtained 1,3-bis(methoxycarbonylaminomethyl) cyclohexane were as follows.
EI-MS (m/z); 258 [M].
CI-MS (m/z); 259 [M+1].
¹H-NMR (CDCl₃,δ (ppm)); 0.52-1.83 (10H, m), 3.00 - 3.14 (4H, m), 3.66 (6H, s), 4.99 (2H, s).
¹³C-NMR (CDCl₃, δ (ppm)); 20.7, 25.2, 29.2, 30.4, 31.9, 33.2, 34.6, 38.0, 45.0, 47.3, 52.0, 157.3, 157.4.
IR(KBr method, cm⁻¹); 664, 781, 837, 891, 910, 925, 1008, 1034, 1053, 1083, 1095, 1150, 1192, 1261, 1312, 1450, 1543, 1701, 2856, 2922, 2940, 3068, 3359.
EA; Calcd: C, 55.80%; H, 8.58%; N, 10.84%
Found: C, 55.53%; H, 8.28%; N, 10.75%.

### Example 10 (Synthesis of m-xylylenedimethyl carbamate)

In a vessel made of glass having an internal volume of about 19 ml and being equipped with a stirring device, a thermometer and a condenser were charged 500 mg (3.67 mmol) of m-xylylenediamine, 1.98 g (22.03 mmol) of dimethyl carbonate, 5 mL of toluene, and 25 mg of lipase from *Candida Antarctica* (Novozym 435 (trade name), manufactured by Novozymes) and mixed, and the mixture was reacted under stirring at 70°C for 60 hours. After completion of the reaction, the reaction solution was filtered, and the filtrate was washed with xylene. The filtrate solution was concentrated to obtain 908 mg of m-xylylenedimethyl carbamate in the form of white solids (yield of the isolated product, based on m-xylylenediamine: 98%).
The obtained m-xylylenedimethyl carbamate had the following values of physical properties.
EI-MS (m/z); 252 [M].
CI-MS (m/z); 253 [M+1].
¹H-NMR(CDCl₃, δ (ppm)); 0.94 (3H, t, J = 7.32Hz), 1.40 (2H, tq, J = 7.32, Hz), 1.66 (2H, tt, J = 6.59, Hz), 3.78 (3H, s), 4.14 (2H, t, J = 6.59Hz).
¹³C-NMR (CDCl₃, δ (ppm)); 45.0, 52.3, 126.6, 129.0, 139.1, 157.1.
IR (KBr method, cm⁻¹); 434, 542, 558, 579, 671, 689, 789, 895, 917, 941, 1006, 1048, 1089, 1140, 1170, 1197, 1255, 1299, 1314, 1353, 1371, 1448, 1463, 1494, 1529, 1548, 1612, 1695, 2789, 2846, 2878, 2948, 3038, 3066, 3324.
EA; Calcd: C, 57.03%; H, 6.39%; N, 11.10%
Found: C, 57.22%; H, 6.20%; N, 11.09%.

### Example 11 (Synthesis of isophoronedimethyl carbamate)

In a vessel made of glass having an internal volume of about 19 ml and being equipped with a stirring device, a thermometer and a condenser were charged 500 mg (3.67 mmol) of isophoronediamine, 1.98 g (22.03 mmol) of dimethyl carbonate, 5 mL of toluene, and 25 mg of lipase from *Candida Antarctica* (Novozym 435 (trade name), manufactured by Novozymes) and mixed, and the mixture was reacted under stirring at 70°C for 120 hours. 75 mg of lipase from *Candida Antarctica* (Novozym 435 (trade name), manufactured by Novozymes) was further added to the mixture, and the mixture was reacted under stirring at 70°C for 400 hours. After completion of the reaction, the reaction solution was filtered, and the filtrate was washed with toluene. The filtrate solution was concentrated to obtain 807 mg of isophoronedimethyl carbamate in the form of white solids (yield of the isolated product, based on isophoronediamine: 96%).
Physical properties of the obtained isophoronedimethyl carbamate were as follows.
EI-MS (m/z); 286 [M].
CI-MS (m/z); 287 [M+1].
¹H-NMR (CDCl₃, δ (ppm)); 0.81 - 1.22 (13H, m), 1.67 - 1.75 (2H, m), 2.91 (1H, s), 2.94 (1H, s), 3.65 - 3.66 (6H, m), 3.79 (1H, s), 4.61 (1H, s), 4.90 (1H, s).
IR (Liquid membrane method, cm⁻¹); 668, 738, 779, 866, 893, 954, 1017, 1043, 1069, 1139, 1154, 1193, 1251, 1311, 1343, 1365, 1387, 1463, 1542, 1696, 2848, 2922, 2954, 3069, 3333,

### Example 12 (Synthesis of 1,3-bis(methoxycarbonylaminomethyl)cyclohexane)

In a vessel made of glass having an internal volume of about 19 ml and being equipped with a stirring device, a thermometer and a condenser were charged 500 mg (3.52 mmol) of 1,3-bis(aminomethyl)cyclohexane, 1.90 g (21.09 mmol) of dimethyl carbonate, 5 mL of toluene, and 25 mg of lipase from *Candida Antarctica* (Novozym 435 (trade name), manufactured by Novozymes) and mixed, and the mixture was reacted under stirring at 70°C for 48 hours. After completion of the reaction, a portion of the reaction solution was taken and quantitative determination (internal standard method) was carried out by using high performance liquid chromatography. The results showed that the reaction solution contained 899 mg of 1,3-bis(methoxycarbonylaminomethyl) cyclohexane as a product (yield based on 1,3-bis(aminomethyl)eyclohexane: 99%).
Physical properties of the obtained 1,3-bis(methoxycarbonylaminomethyl) cyclohexane were as follows.
EI-MS (m/z); 258 [M].
CI-MS (m/z); 259 [M+1].
¹H-NMR (CDCl₃, δ (ppm)); 0.52 - 1.83 (10H, m), 3.00-3.14 (4H, m), 3.66(6H, s), 4.99 (2H, s).
¹³C-NMR (CDCl₃, δ (ppm)); 20.7, 25.2, 29.2, 30.4, 31.9, 33.2, 34.6, 38.0, 45.0, 47.3, 52.0, 157.3, 157.4.
IR (KBr method, cm⁻¹); 664, 781, 837, 891, 910, 925, 1008, 1034, 1053, 1083, 1095, 1150, 1192, 1261, 1312, 1450, 1543, 1701, 2856, 2922, 2940, 3068, 3359.
EA; Calcd: C, 55.80%; H, 8.58%; N, 10.84%
Found: C, 55.53%; H, 8.28%; N, 10.75%.

### Example 13 (Synthesis of 1,3-bis(methoxycarbonylaminomethyl)cyclohexane)

In a vessel made of glass having an internal volume of about 19 ml and being equipped with a stirring device, a thermometer and a condenser were charged 500 mg (3.52 mmol) of 1,3-bis(aminomethyl)cyclohexane, 1.90 g (21.09 mmol) of dimethyl carbonate, 5 mL of toluene, and 25 mg of lipase from *Candida Antarctica* (Novozym 435 (trade name), manufactured by Novozymes) and mixed, and the mixture was reacted under stirring at 65°C for 48 hours. After completion of the reaction, a portion of the reaction solution was taken and quantitative determination (internal standard method) was carried out by using high performance liquid chromatography. The results showed that the reaction solution contained 899 mg of 1,3-bis(methoxycarbonylaminomethyl)cyclohexane as a product (yield based on 1,3-bis(aminomethyl)cyclohexane: 99%).
The obtained 1,3-bis(methoxycarbonylaminomethyl)cyclohexane had the following values of physical properties.
EI-MS (m/z); 258 [M].
CI-MS (m/z); 259 [M+1].
¹H-NMR (CDCl₃, δ(ppm)); 0.52 - 1.83 (10H, m), 3.00 - 3.14 (4H, m), 3.66 (6H, s), 4.99 (2H, s).
¹³C-NMR (CDCl₃, δ(ppm)); 20.7, 25.2, 29.2, 30.4, 31.9, 33.2, 34.6, 38.0, 45.0, 47.3, 52.0, 157.3, 157.4.
IR (KBr method, cm⁻¹); 664, 781, 837, 891, 910, 925, 1008, 1034, 1053, 1083, 1095, 1150, 1192, 1261, 1312, 1450, 1543, 1701, 2856, 2922, 2940, 3068, 3359.
EA; Calcd: C, 55.80%; H, 8.58%; N, 10.84%
Found: C, 55.53%; H, 8.28%; N, 10.75%.

### Example 14 (Synthesis of 1,3-bis(methoxycarbonylaminomethyl)cyclohexane)

In a vessel made of glass having an internal volume of about 19 ml and being equipped with a stirring device, a thermometer and a condenser were charged 500 mg (3.52 mmol) of 1,3-bis(aminomethyl)cyclohexane, 1.90 g (21.09 mmol) of dimethyl carbonate, 5 mL of toluene, and 25 mg of lipase from *Candida Antarctica* (Novozym 435 (trade name), manufactured by Novozymes) and mixed, and the mixture was reacted under stirring at 60°C for 48 hours. After completion of the reaction, a portion of the resultant reaction solution was taken and quantitative determination (internal standard method) was carried out by using high performance liquid chromatography. The results showed that the reaction solution contained 772 mg of 1,3-bis(methoxycarbonylaminomethyl) cyclohexane as a product (yield based on 1,3-bis(aminomethyl)cyclohexane: 85%).
Physical properties of the obtained 1,3-bis(methoxycarbonylaminomethyl) cyclohexane were as follows.
EI-MS (m/z); 258 [M].
CI-MS (m/z); 259 [M+1].
¹H-NMR (CDCl₃, δ (ppm)); 0.52 - 1.83 (10H, m), 3.00 - 3.14 (4H, m), 3.66 (6H, s), 4.99 (2H,s).
¹³C-NMR (CDCl₃, µ (ppm)); 20.7, 25.2, 29.2, 30.4, 31.9, 33.2, 34.6, 38.0, 45.0, 47.3, 52.0, 157.3, 157.4.
IR (KBr method, cm⁻¹); 664, 781, 837, 891, 910, 925, 1008, 1034, 1053, 1083, 1095, 1150, 1192, 1261, 1312, 1450, 1543, 1701, 2856, 2922, 2940, 3068, 3359.
EA; Calcd: C, 55.80%; H, 8.58%; N, 10.84%
Found: C, 55.53%; H, 8.28%; N, 10.75%.

### Example 15 (Synthesis of 1,3-bis(methoxycarbonylaminomethyl)cyclohexane)

In a vessel made of glass having an internal volume of about 19 ml and being equipped with a stirring device, a thermometer and a condenser were charged 500 mg (3.52 mmol) of 1,3-bis(aminomethyl)cyclohexane, 1.90 g (21.09 mmol) of dimethyl carbonate, 5 mL of cyclohexane, and 25 mg of lipase from *Candida Antarctica* (Novozym 435 (trade name), manufactured by Novozymes) and mixed, and the mixture was reacted under stirring at 70°C for 48 hours. After completion of the reaction, a portion of the reaction solution was taken and quantitative determination (internal standard method) was carried out by using high performance liquid chromatography. The results showed that the reaction solution contained 899 mg of 1,3-bis(methoxycarbonylaminomethyl) cyclohexane as a product (yield based on 1,3-bis(aminomethyl)cyclohexane: 99%).
Physical properties of the obtained 1,3-bis(methoxycarbonylaminomethyl) cyclohexane were as follows.
EI-MS (m/z); 258 [M].
CI-MS (m/z); 259 [M+1].
¹H-NMR (CDCl₃, δ (ppm)); 0.52 - 1.83 (10H, m), 3.00 - 3.14 (4H, m), 3.66 (6H, s), 4.99 (2H, s).
¹³C-NMR (CDCl₃, δ (ppm)); 20.7, 25.2, 29.2, 30.4, 31.9, 33.2, 34.6, 38.0, 45.0, 47.3, 52.0, 157.3, 157.4.
IR (KBr method, cm⁻¹); 664, 781, 837, 891, 910, 925, 1008, 1034, 1053, 1083, 1095, 1150, 1192, 1261, 1312, 1450, 1543, 1701, 2856, 2922, 2940, 3068, 3359.
EA; Calcd: C, 55.80%; H, 8.58%; N,10.84%
Found: C, 55.53%; H, 8.28%; N,10.75%.

### Example 16 (Synthesis of 1 ,3-bis(methoxycarbonylaminomethyl)cydohexane)

In a vessel made of glass having an internal volume of about 19 ml and being equipped with a stirring device, a thermometer and a condenser were charged 500 mg (3.52 mmol) of 1,3-bis(aminomethyl)cyclohexane, 1.90 g (21.09 mmol) of dimethyl carbonate, 5 mL of diisopropyl ether, and 25 mg of lipase from *Candida Antarctica* (Novozym 435 (trade name), manufactured by Novozymes) and mixed, and the mixture was reacted under stirring at 70°C for 48 hours. After completion of the reaction, a portion of the reaction solution was taken and quantitative determination (internal standard method) was carried out by using high performance liquid chromatography. The results showed that the reaction solution contained 899 mg of 1,3-bis(methoxycarbonylaminomethyl) cyclohexane as a product (yield based on 1,3-bis(aminomethyl)cyclohexane: 99%).
Physical properties of the obtained 1,3-bis(methoxycarbonylaminomethyl) cyclohexane were as follows.
EI-MS (m/z); 258 [M].
CI-MS (m/z); 259 [M+1].
¹H-NMR (CDCl₃, δ (ppm)); 0.52 - 1.83 (10H, m), 3.00 - 3.14 (4H, m), 3.66 (6H, s), 4.99 (2H, s).
¹³C-NMR (CDCl₃, δ (ppm)); 20.7, 25.2, 29.2, 30.4, 31.9, 33.2, 34.6, 38.0, 45.0, 47.3, 52.0, 157.3, 157.4.
IR (KBr method, cm⁻¹); 664, 781, 837, 891, 910, 925, 1008, 1034, 1053, 1083, 1095, 1150, 1192, 1261, 1312, 1450, 1543, 1701, 2856, 2922, 2940, 3068, 3359.
EA; Calcd: C, 55.80%; H, 8.58%; N, 10.84%
Found: C, 55.53%; H, 8.28%; N, 10.75%.

### Example 17 ((Synthesis of 1,3-bis(methoxycarbonylaminomethyl)cyclohexane))

7.30 g of lipase from *Candida Antarctica* (Novozym 435 (trade name), manufactured by Novozymes) was swollen with toluene, and a moderate pressure glass column (300 m/m) being equipped with a jacket and being connected to a microserumpump (Model MSP-101-00, manufactured by YAMAZEN) was packed with the resultant lipase. After the immobilized enzyme packing, warm water at 70°C was fed into the jacket of the glass column, and, while maintaining the inside of the glass column at 70°C, a mixture solution of 91.1 g of 1,3-bis(aminomethyl)cyclohexane, 345.6 g of dimethyl carbonate, and 908 g of toluene was fed to the glass column at a rate of 24 ml per hour from the top of the glass column, and the reaction solution which had passed through the glass column was recovered from the bottom of the glass column. After the recovery, the reaction solution was concentrated to obtain a product in the form of white solids (yield based on 1,3-bis(aminomethyl)cyclohexane: 95% (quantitative determination by high performance liquid chromatography)). Further, the initial STY (space time yield) in the fixed bed flow system was 81.5 (g/L-cat/h). As the reaction proceeded, the substrate solution was added at appropriate times so that the ratio became the same as the initial ratio. At a point in time that the feeding of the solution was continued for 60 hours, the STY was 80.8 and the yield was 94.2%.

### Example 18 (Synthesis of 1,8-octamethylenedimethyl carbamate)

In a vessel made of glass having an internal volume of about 19 ml and being equipped with a stirring device, a thermometer and a condenser were charged 500 mg (3.47 mmol) of 1,8-octanediamine, 1.87 g (20.80 mmol) of dimethyl carbonate, 5 mL of toluene, and 25 mg of lipase from *Candida Antarctica* (Novozym 435 (trade name), manufactured by Novozymes) and mixed, and the mixture was reacted under stirring at 70°C for 48 hours. After completion of the reaction, the reaction solution was filtered, and the filtrate was washed with toluene. The filtrate solution was concentrated to obtain 785 mg of 1,8-octamethylenedimethyl carbamate in the form of white solids (yield of the isolated product, based on 1,8-octanediamine: 87%).
Physical properties of the obtained 1,8-octamethylenedimethyl carbamate were as follows.
EI-MS (m/z); 260 [M]
CI-MS (m/z); 261 [M+1]
¹H-NMR (CDCl₃, δ (ppm)); 1.30 (6H, m), 1.48 (4H, tt, J = 6.35, 6.35Hz), 3.12 - 3.19 (4H, m), 3.66 (6H, s), 4.77 (2H, s)
¹³C-NMR (CDC1₃, δ (ppm)); 26.6, 29.1, 30.0, 41.0, 52.0, 157.1
IR(KBr method, cm⁻¹); 488, 548, 624, 708, 730, 759, 785, 938, 986, 1037, 1062, 1080, 1142, 1197, 1211, 1260, 1308, 1363, 1436, 1464, 1479, 1531, 1688, 2854, 2873, 2924, 2942,3044,3342
Elemental analysis; Calcd: C, 55.36%; H, 9.29%; N, 10.76%
Found: C, 55.30%; H, 9.12%; N, 10.72%

### Examples 19 to 23 (Synthesis of dicarbamate compound)

Reactions were conducted in the same manner as in Example 18 except that the type of the diamine compound and the reaction time were changed and the mol of the diamine compound employed in Example 18 was not changed. The results are shown in Table 1.

**[Table 1]**

| Synthesis of dicarbamate using various diamines | | | | | |
|---|---|---|---|---|---|
| | Diamine compound | Number of carbon atoms of main chain of Z | Total number of carbon atoms of Z | Reaction time (hour) | Quantitative yield (%) by HPLC |
| Example 18 | 1,8-Octanediamine | 6 | 6 | 48 | 87 |
| Example 19 | 1,9-Nonanediamine | 7 | 7 | 48 | 98 |
| Example 20 | 1,10-Decanediamine | 8 | 8 | 48 | 99 |
| Example 21 | 1,12-Dodecanediamine | 10 | 10 | 48 | 99 |
| Example 22 | 1,8-Octanediamine | 6 | 6 | 60 | 99 |
| Example 23 | 1,12-Dodecanediamine | 10 | 10 | 19 | 98 |

### Example 24 (Synthesis of 1,3-bis(methoxycarbonylaminomethyl)cyclohexane)

In a vessel made of glass having an internal volume of about 19 ml and being equipped with a stirring device, a thermometer and a condenser were charged 400 mg (2.81 mmol) of 1,3-bis(aminomethyl)cyclohexane, 3.04 g (33.75 mmol) of dimethyl carbonate, 4 mL of toluene, and 20 mg of lipase from *Candida Antarctica* (Novozym 435 (trade name), manufactured by Novozymes), and mixed, and the mixture was reacted under stirring at 70°C for 22 hours. After 22 hours, the reaction solution was taken and quantitative determination of a product (internal standard method) was carried out by high performance liquid chromatography. The results showed that 697 mg of 1,3-bis(methoxycarbonylaminomethyl)cyclohexane was contained (quantitative yield based on 1,3-bis(aminomethyl)cyclohexane: 96%).

### Example 25 (Synthesis of 1,3-bis(methoxycarbonylaminomethyl)cyclohexane)

In a vessel made of glass having an internal volume of about 19 ml and being equipped with a stirring device, a thermometer and a condenser were charged 400 mg (2.81 mmol) of 1,3-bis(aminomethyl)cyclohexane, 2.53 g (28.12 mmol) of dimethyl carbonate, 4 mL of toluene, and 8 mg of lipase from *Candida Antarctica* (Novozym 435 (trade name), manufactured by Novozymes) and mixed, and the mixture was reacted under stirring at 70°C for 48 hours. After 48 hours, the resultant reaction solution was taken and quantitative determination (internal standard method) was carried out by using high performance liquid chromatography. The results showed that 712 mg of 1,3-bis(methoxycarbonylaminomethyl)cyclohexane was contained (quantitative yield based on 1,3-bis(aminomethyl)cyclohexane: 98%).

### Example 26 (Synthesis of 1,3-bis(methoxycarbonylaminomethyl)cyclohexane)

In a vessel made of glass having an internal volume of about 19 ml and being equipped with a stirring device, a thermometer and a condenser were charged 500 mg (3.52 mmol) of 1,3-bis(aminomethyl)cyclohexane, 2.67 g (29.67 mmol) of dimethyl carbonate, 2.5 mL of toluene, and 25 mg of lipase from *Candida Antarctica* (Novozym 435 (trade name), manufactured by Novozymes) and mixed, and the mixture was reacted under stirring at 70°C for 15.5 hours. After 22 hours, the resultant reaction solution was taken and quantitative determination (internal standard method) was carried out by using high performance liquid chromatography. The results showed that 863 mg of 1,3-bis(methoxycarbonylaminomethyl)cyclohexane was contained (quantitative yield based on 1,3-bis(aminomethyl)cyclohexane: 95%).

### Example 27 (Synthesis of m-xylylenedimethyl carbamate)

In a vessel made of glass having an internal volume of about 19 ml and being equipped with a stirring device, a thermometer and a condenser were charged 500 mg (3.67 mmol) of m-xylylenediamine, 3.31 g (36.71 mmol) of dimethyl carbonate, 3.5 mL of toluene, and 10 mg of lipase from *Candida Antarctica* (Novozym 435 (trade name), manufactured by Novozymes) and mixed, and the mixture was reacted under stirring at 70°C for 42 hours. After 42 hours, the resultant reaction solution was taken and quantitative determination (internal standard method) was carried out by using high performance liquid chromatography. The results showed that 868 mg of m-xylylenedimethyl carbamate was contained (quantitative yield based on m-xylylenediamine: 94%).
Physical properties of the obtained m-xylylenedimethyl carbamate were the same as those shown in Example 10.

### Example 28 (Synthesis of m-xylylenedimethyl carbamate)

In a vessel made of glass having an internal volume of about 300 ml and being equipped with a stirring device, a thermometer and a condenser were charged 10.00 g (73.43 mmol) of m-xylylenediamine, 39.69 g (440.56 mmol) of dimethyl carbonate, 150 mL of xylene, and 250 mg of lipase from *Candida Antarctica* (Novozym 435 (trade name), manufactured by Novozymes) and mixed, and the mixture was reacted under stirring at 70°C for 48 hours. After completion of the reaction, 50 mL of methanol was added to the resultant reaction mixture followed by filtration, and the filtrate was washed with methanol. The filtrate solution was concentrated to obtain 17.62 g of m-xylylenedimethyl carbamate in the form of white solids (yield of the isolated product, based on m-xylylenediamine: 95%).
Physical properties of the obtained m-xylylenedimethyl carbamate were the same as those shown in Example 10.

### Example 29 (Synthesis of 1,10-decamethylenedimethyl carbamate)

In a vessel made of glass having an internal volume of about 100 ml and being equipped with a stirring device, a thermometer and a condenser were charged 4.0 g (23.21 mmol) of 1,10-decanediamine, 12.55 g (139.28 mmol) of dimethyl carbonate, 40 mL of toluene, and 100 mg of lipase from *Candida Antarctica* (Novozym 435 (trade name), manufactured by Novozymes) and mixed, and the mixture was reacted under stirring at 70°C for 48 hours. After completion of the reaction, 40 mL of methanol was added to the resultant reaction mixture followed by filtration, and the filtrate was washed with methanol. The filtrate solution was concentrated to obtain 6.43 g of 1,10-decamethylenedimethyl carbamate in the form of white solids (yield of the isolated product, based on 1,10-decanediamine: 96%).
Physical properties of the obtained 1,10-decamethylenedimethyl carbamate were as follows.
EI-MS (m/z); 288 [M].
CI-MS (m/z); 289 [M+1].
¹H-NMR (CDCl₃, δ (ppm)); 1.27 (10H, m), 1.48 (4H, tt, J = 6.84, 6.84Hz), 3.13 - 3.19 (4H, m), 3.66 (6H, s), 4.71 (2H, s). ¹³ C-NMR (CDCl₃, δ (ppm)); 26.7, 29.2, 29.4, 30.0, 41.1, 52.0, 157.1.
IR (KBr method, cm-¹); 484, 556, 623, 709, 727, 781, 937, 988, 1011, 1049, 1064, 1090, 1142, 1201, 1249, 1284, 1339, 1371, 1435, 1464, 1479, 1527, 1690, 2853, 2873, 2924,2943,3042,3346.
EA; Calcd: C, 58.31%; H, 9.79%; N, 9.71 %
Found: C, 58.15%; H, 9.53%; N, 9.56%.

### Example 30 (Synthesis of 1,12-dodecamethylenedimethyl carbamate)

In a vessel made of glass having an internal volume of about 300 ml and being equipped with a stirring device, a thermometer and a condenser were charged 10 g (49.91 mmol) of 1,12-dodecanediamine, 26.98 g (299.46 mmol) of dimethyl carbonate, 100 mL of toluene, and 250 mg of lipase from *Candida Antarctica* (Novozym 435 (trade name), manufactured by Novozymes) and mixed, and the mixture was reacted under stirring at 70°C for 48 hours. After completion of the reaction, 100 mL of methanol was added to the resultant reaction mixture, followed by filtration, and the filtrate was washed using methanol. The filtrate solution was concentrated to obtain 15.48 g of 1,12-dodecamethylenedimethyl carbamate in the form of white solids (yield of the isolated product, based on 1,12-dodecanediamine: 98%).
Physical properties of the obtained 1,12-dodecamethylenedimethyl carbamate were the same as those shown in Example 8.

### Comparative Example 1 (Synthesis of 1,3-bis(methoxycarbonylaminomethyl)cyclohexane)

In a vessel made of glass having an internal volume of about 19 ml and being equipped with a stirring device, a thermometer and a condenser were charged 500 mg (3.52 mmol) of 1,3-bis(aminomethyl)cyclohexane, 1.90 g (21.09 mmol) of dimethyl carbonate, 5 mL of n-hexane, and 25 mg of lipase from *Candida Antarctica* (Novozym 435 (trade name), manufactured by Novozymes) and mixed, and the mixture was reacted under stirring at 70°C for 72 hours. After completion of the reaction, a portion of the resultant reaction solution was taken out and quantitative determination (internal standard method) was carried out by using high performance liquid chromatography. The results showed that the reaction solution contained 209 mg of 1,3-bis(methoxycarbonylaminomethyl)cyclohexane as a product (yield based on 1,3-bis(aminomethyl)cyclohexane: 23%).
Physical properties of the obtained 1,3-bis(methoxycarbonylaminomethyl) cyclohexane were as follows.
EI-MS (m/z); 258 [M].
CI-MS (m/z); 259 [M+1].
¹H-NMR (CDCl₃, δ (ppm)); 0.52 - 1.83 (10H, m), 3.00 - 3.14 (4H, m), 3.66 (6H, s), 4.99 (2H, s).
¹³C-NMR (CDCl₃, δ (ppm)); 20.7, 25.2, 29.2, 30.4, 31.9, 33.2, 34.6, 38.0, 45.0, 47.3, 52.0, 157.3, 157.4.
IR (KBr methods, cm-¹); 664, 781, 837, 891, 910, 925, 1008, 1034, 1053, 1083, 1095, 1150, 1192, 1261, 1312, 1450, 1543, 1701, 2856, 2922, 2940, 3068, 3359.
EA; Calcd: C, 55.80%; H, 8.58%; N, 10.84%
Found: C, 55.53%; H, 8.28%; N, 10.75%.

### Comparative Example 2 (Synthesis of 1,3-bis(methoxycarbonylaminomethyl) cyclohexane)

In a vessel made of glass having an internal volume of about 19 ml and being equipped with a stirring device, a thermometer and a condenser were charged 500 mg (3.52 mmol) of 1,3-bis(aminomethyl)cyclohexane, 1.90 g (21.09 mmol) of dimethyl carbonate, 5 mL of n-heptane, and 25 mg of lipase from *Candida Antarctica* (Novozym 435 (trade name), manufactured by Novozymes) and mixed, and the mixture was reacted under stirring at 70°C for 72 hours. After completion of the reaction, a portion of the resultant reaction solution was taken out and subjected to quantitative determination (internal standard method) was carried out by using high performance liquid chromatography. The results showed that the reaction solution contained 245 mg of 1,3-bis(methoxycarbonylaminomethyl) cyclohexane as a product (yield based on 1,3-bis(aminomethyl)cyclohexane: 27%).
Physical properties of the obtained 1,3-bis(methoxycarbonylaminomethyl) cyclohexane were as follows.
EI-MS (m/z); 258 [M].
CI-MS (m/z); 259 [M+1].
¹H-NMR (CDCl₃, δ (ppm)); 0.52 - 1.83 (10H, m), 3.00 - 3.14 (4H, m), 3.66 (6H, s), 4.99 (2H, s).
¹³C-NMR (CDCl₃, δ (ppm)); 20.7, 25.2, 29.2, 30.4, 31.9, 33.2, 34.6, 38.0, 45.0, 47.3, 52.0, 157.3, 157.4.
IR(KBr method, cm⁻¹); 664, 781, 837, 891, 910, 925, 1008, 1034, 1053, 1083, 1095, 1150, 1192, 1261, 1312, 1450, 1543, 1701, 2856, 2922, 2940, 3068, 3359.
EA; Calcd: C, 55.80%; H, 8.58%; N, 10.84%
Found: C, 55.53%; H, 8.28%; N, 10.75%.

### Comparative Example 3 (Synthesis of 1,3-bis(methoxycarbonylaminomethyl) cyclohexane)

In a vessel made of glass having an internal volume of about 19 ml and being equipped with a stirring device, a thermometer and a condenser were charged 500 mg (3.52 mmol) of 1,3-bis(aminomethyl)cyclohexane, 1.90 g (21.09 mmol) of dimethyl carbonate, 5 mL of tetrahydrofuran, and 25 mg of lipase from *Candida Antarctica* (Novozym 435 (trade name), manufactured by Novozymes) and mixed, and the mixture was reacted under stirring at 70°C for 72 hours. After completion of the reaction, a portion of the resultant reaction solution was taken out and quantitative determination (internal standard method) was carried out by using high performance liquid chromatography. The results showed that the reaction solution contained 554 mg of 1,3-bis(methoxycarbonylaminomethyl)cyclohexane as a product (yield based on 1,3-bis(aminomethyl)cyclohexane: 61 %).
Physical properties of the obtained 1,3-bis(methoxycarbonylaminomethyl) cyclohexane were as follows.
EI-MS (m/z); 258 [M].
CI-MS (m/z); 259 [M+1].
¹H-NMR (CDCl₃, δ (ppm)); 0.52 - 1.83(10H, m), 3.00 - 3.14 (4H, m), 3.66 (6H, s), 4.99 (2H, s).
¹³C-NMR (CDCl₃, δ (ppm)); 20.7, 25.2, 29.2, 30.4, 31.9, 33.2, 34.6, 38.0, 45.0, 47.3, 52.0, 157.3, 157.4.
IR (KBr method, cm⁻¹); 664, 781, 837, 891, 910, 925, 1008, 1034, 1053, 1083, 1095, 1150, 1192, 1261, 1312, 1450, 1543, 1701, 2856, 2922, 2940, 3068, 3359.
EA; Calcd: C, 55.80%; H, 8.58%; N, 10.84%
Found: C, 55.53%; H, 8.28%; N, 10.75%.

### Comparative Example 4 (Synthesis of 1,3-bis(methoxycarbonylaminomethyl) cyclohexane)

In a vessel made of glass having an internal volume of about 19 ml and being equipped with a stirring device, a thermometer and a condenser were charged 500 mg (3.52 mmol) of 1,3-bis(aminomethyl)cyclohexane, 1.90 g (21.09 mmol) of dimethyl carbonate, and 25 mg of lipase from *Candida Antarctica* (Novozym 435 (trade name), manufactured by Novozymes) and mixed, and the mixture was reacted under stirring at 50°C for 48 hours. After completion of the reaction, a portion of the resultant reaction solution was taken out and quantitative determination (internal standard method) was carried out by using high performance liquid chromatography. The results showed that the reaction solution contained 191 mg of 1,3-bis(methoxycarbonylaminomethyl)cyclohexane as a product (yield based on 1,3-bis(aminomethyl)cyclohexane: 21%).
Physical properties of the obtained 1,3-bis(methoxycarbonylaminomethyl) cyclohexane were as follows.
EI-MS (m/z); 258 [M].
CI-MS (m/z); 259 [M+1].
¹H-NMR (CDCl₃, δ (ppm)); 0.52 - 1.83 (10H, m), 3.00 - 3.14 (4H, m), 3.66 (6H, s), 4.99 (2H, s).
¹³C-NMR (CDCl₃, δ(ppm)); 20.7, 25.2, 29.2, 30.4, 31.9, 33.2, 34.6, 38.0, 45.0, 47.3, 52.0, 157.3, 157.4.
IR (KBr method, cm⁻¹); 664, 781, 837, 891, 910, 925, 1008, 1034, 1053, 1083, 1095, 1150, 1192, 1261, 1312, 1450, 1543, 1701, 2856, 2922, 2940, 3068, 3359.
EA; Calcd: C, 55.80%; H, 8.58%; N, 10.84%
Found: C, 55.53%; H, 8.28%; N, 10.75%.

### Industrial Applicability

The present invention relates to a process for obtaining a carbamate compound from an amine compound and a carbonate compound. The carbamate compound obtained by the process of the present invention is useful, for example, as a raw material in the production of an isocyanate without using toxic phosgene.

## Claims

1. A process for preparing a carbamate compound which comprises the step of reacting an amine compound which has at least one amino group per molecule wherein the amine compound is selected from the group consisting of an aliphatic amine which may be substituted by an alicyclic group or an aromatic group or which may be interrupted by an alicyclic group or an aromatic group, and an alicyclic amine which may be substituted by an aliphatic group,
with a carbonate compound
in the presence of at least one organic solvent selected from the group consisting of a saturated cyclic hydrocarbon, an unsaturated cyclic hydrocarbon, and a non-cyclic ether by using a hydrolase.

2. The process for preparing a carbamate compound according to claim 1, wherein the organic solvent is at least one organic solvent selected from the group consisting of an unsubstituted cycloalkyl, an aromatic hydrocarbon and a dialkyl ether.

3. The process for preparing a carbamate compound according to claim 1 or 2, wherein the hydrolase is immobilized on a support.

4. The process for preparing a carbamate compound according to claim 3, wherein the hydrolase is a hydrolase immobilized on a support which is incorporated as a fixed bed to the inner side of a reaction vessel, and the reaction is a reaction comprising the step of allowing the amine compound and the carbonate compound to pass through the reaction vessel.

5. The process for preparing a carbamate compound according to any one of claims 1 to 4, wherein the hydrolase is a lipase.

6. The process for preparing a carbamate compound according to any one of claims 1 to 5, wherein the amine compound is a monoamine compound represented by the formula (1):
R¹—(CH₂)ₙ—NH₂ (1)
wherein R¹ is a linear or branched alkyl group having 1 to 20 carbon atoms, a linear or branched alkenyl group having 2 to 20 carbon atoms, a linear or branched alkynyl group having 2 to 20 carbon atoms, a cycloalkylalkyl group having 4 to 24 carbon atoms, an aralkyl group having 7 to 21 carbon atoms, or a cycloalkyl group having 3 to 20 carbon atoms, each of which may have a substituent, and
n is 0 or 1.

7. The process for preparing a carbamate compound according to any one of claims 1 to 5, wherein the amine compound is a diamine compound represented by the formula (4):
H₂N—(CH₂)m—R³—(CH₂)ₚ—NH₂ (4)
wherein R³ is a linear or branched alkylene group having 1 to 30 carbon atoms, a (linear alkylene having 1 to 4 carbon atoms)-(cycloalkylene having 3 to 20 carbon atoms)-(linear alkylene having 1 to 4 carbon atoms) group, a (linear alkylene having 1 to 4 carbon atoms)-(arylene having 6 to 20 carbon atoms)-(linear alkylene having 1 to 4 carbon atoms) group, a cycloalkylene group having 3 to 20 carbon atoms, or a (linear alkylene having 1 to 4 carbon atoms)-(cycloalkylene having 3 to 20 carbon atoms) group, each of which may have a substituent, and
m and p are independently from each other 0 or 1.

8. The process for preparing a carbamate compound according to claim 7, wherein the diamine compound is at least one selected from the group consisting of 1,3-bis(aminomethylcyclohexane), 1,4-bis(aminomethylcyclohexane), 2,5-bis(aminomethyl)bicyclo[2.2.1]heptane, 2,6-bis(aminomethyl)bicyclo[2.2.1]heptane, 1,3-bis(aminomethyl)benzene, and 1,4-bis(aminomethyl)benzene.

9. The process for preparing a carbamate compound according to any one of claims 1 to 5 and 7, wherein the amine compound is a diamine compound represented by the formula (4a):
H₂NH₂C—Z—CH₂NH₂ (4a)
wherein Z is a linear or branched alkylene group which has a main chain having 6 or more carbon atoms and may have a substituent.

10. The process for preparing a carbamate compound according to claim 9, wherein Z is a linear or branched alkylene group having a main chain having 6 to 18 carbon atoms, and having 6 to 22 carbon atoms in total.

11. The process for preparing a carbamate compound according to claim 9 or 10, wherein the diamine compound represented by the formula (4a) is at least one diamine compound selected from the group consisting of 1,8-octanediamine, 1,9-nonanediamine, 1,10-decanediamine, and 1,12-dodecanediamine.

12. The process for preparing a carbamate compound according to any one of claims 1 to 11, wherein the reaction temperature is 55 to 90°C.
